# EUROPEAN PATENT SPECIFICATION

(11) **EP 3 624 838 B1**
(45) Date of publication and mention of the grant of the patent: **25.01.2023**
(21) Application number: 18801838.6
(22) Date of filing: 15.05.2018
(51) Int. Cl.: A61K 38/17, A61K 48/00, A61P 25/28, C07K 14/705, A61K 47/68, A61P 25/16

(54) **METHODS OF USE OF SOLUBLE CD24 FOR NEUROPROTECTION AND REMYELINATION**
VERFAHREN ZUR VERWENDUNG VON LÖSLICHEM CD24 FÜR NEUROPROTEKTION UND REMYELINISIERUNG
PROCÉDÉS D'UTILISATION DE CD24 SOLUBLE POUR LA NEUROPROTECTION ET LA REMYÉLINISATION

(30) Priority: 15.05.2017 US 201762506135 P
(43) Date of publication of application: 25.03.2020
(73) Proprietor: Oncoimmune, Inc., Rockville, Maryland 20850 (US); Children's National Medical Center, Washington, DC 20010 (US)
(72) Inventor: LIU, Yang, Washington District of Columbia 20002 (US); ZHENG, Pan, Washington District of Columbia 20002 (US); LI, Ning, Vestal New York 13850-2873 (US); DEVENPORT, Martin, Gaithersburg Maryland 20878 (US)
(74) Representative: Zacco Denmark A/S
(86) International application number: PCT/US2018/032699
(87) International publication number: WO 2018/213266

(56) References cited:
- US-A1- 2012 294 884
- US-A1- 2015 239 953
- BAI X-F ET AL: "THE HEAT-STABLE ANTIGEN DETERMINES PATHOGENICITY OF SELF-REACTIVE T CELLS IN EXPERIMENTAL AUTOIMMUNE ENCEPHALOMYELITIS", THE JOURNAL OF CLINICAL INVESTIGATION, B M J GROUP, GB, vol. 105, no. 9, 1 May 2000 (2000-05-01), pages 1227-1232, XP002944025, ISSN: 0021-9738, DOI: 10.1172/JCI9012
- DENDROU et al.: "Immunopathology of multiple sclerosis", Nature Reviews Immunology, vol. 15, no. 9, 7 August 2015 (2015-08-07) , pages 545-558, XP055546449,

## Description

### FIELD OF THE INVENTION

The present invention relates to compositions and their use in methods of protecting and maintaining oligodendrocytes, and of treating demyelinating disorders.

### BACKGROUND OF THE INVENTION

Myelin sheaths are layered, lipid-rich structures that wrap around axons to mediate salutatory signal conduction along them and provide them with trophic support. In the central nervous system (CNS), myelin sheaths are formed by oligodendrocytes. Myelin is a target of demyelinating diseases, including neuro-immune disorders such as multiple sclerosis (MS). Remyelination is the process of coaxing oligodendrocyte precursor cells (OPCs) to form oligodendrocytes, cells able to produce new myelin sheaths on demyelinated axons in the central nervous system (CNS). In response to myelin degeneration, multipotent parenchymal progenitor cells called oligodendrocyte progenitor cells (OPCs) are activated and recruited to the damaged areas. This is a process naturally regulated in the body and tends to be very efficient in a healthy CNS. But in people with neuro-immune disorders, remyelination becomes increasingly incomplete, leaving axons permanently demyelinated and vulnerable to degeneration, and in many patients eventually fails, leading to more progressive disease forms.

The reason for remyelination failure in diseases such MS is still not fully understood, and is expected to be complex. Several studies have demonstrated that a proportion of chronically demyelinated MS lesions contains OPCs that fail to differentiate into remyelinating oligodendrocytes. Oligodendrocytes play a critical role in neuroprotection by both remyelination-dependent and -independent mechanisms and confers protection in both inflammatory and degenerative diseases involving the central nervous system, including MS, Alzheimer's diseases and potentially Parkinson's diseases (1-4).

Therefore, there is a large unmet medical need for treatments for demyelinating disorders by promoting the protection and maintenance of oligodendrocytes and promoting myelinating activity.

### SUMMARY OF THE INVENTION

Provided herein are methods of maintaining oligodendrocytes, promoting myelination, and treating demyelinating disorders including inflammatory and degenerative diseases involving the central nervous system. The method may comprise administering a CD24 protein to a subject in need thereof. The demyelinating disorder is selected from Alzheimer's disease, a Parkinson's disease, neuromyelitis optica spectrum disorder, optic neuritis, transverse myelitis, or acute flaccid myelitis. The CD24 protein may maintain oligodendrocytes. The method may further comprise administering another agent that promotes the conversion of oligodendrocytes or promotes myelin production by oligodendrocytes.

The CD24 protein may comprise a mature human CD24. The mature human CD24 may comprise an amino acid sequence set forth in SEQ ID NO: 1 or 2. The CD24 protein may comprise any or all of the extracellular domain of human CD24. The sequence of the CD24 protein may comprise the signal peptide sequence of CD24, which may comprise the amino acid sequence set forth in SEQ ID NO: 4. The signal peptide sequence may allow secretion from a cell expressing the protein. The signal peptide sequence may also be one that is found on another transmembrane or secreted protein, or one modified from an existing signal peptide known in the art. The CD24 protein may be soluble. The CD24 protein may be glycosylated. The CD24 protein may be produced using a eukaryotic protein expression system, which may comprise a vector contained in a Chinese Hamster Ovary cell line or a replication-defective retroviral vector. The replication defective retroviral vector may be stably integrated into the genome of a eukaryotic cell.

The CD24 protein may comprise a protein tag, which may be fused at the N- or C-terminus of the CD24 protein. The protein may comprise a portion of a mammalian immunoglobulin (Ig), which may be a Fc region of a human Ig protein. The human Ig protein may comprise the hinge region and CH2 and CH3 domains of the human Ig protein, and the human Ig protein may be IgG1, IgG2, IgG3, IgG4, or IgA. The Fc region may also comprise the hinge region and CH2, CH3, and CH4 domains of IgM. The CD24 protein may comprise an amino acid sequence set forth in SEQ ID NO: 5, 6, 8, 9, 11, or 12.

### BRIEF DESCRIPTION OF THE DRAWINGS

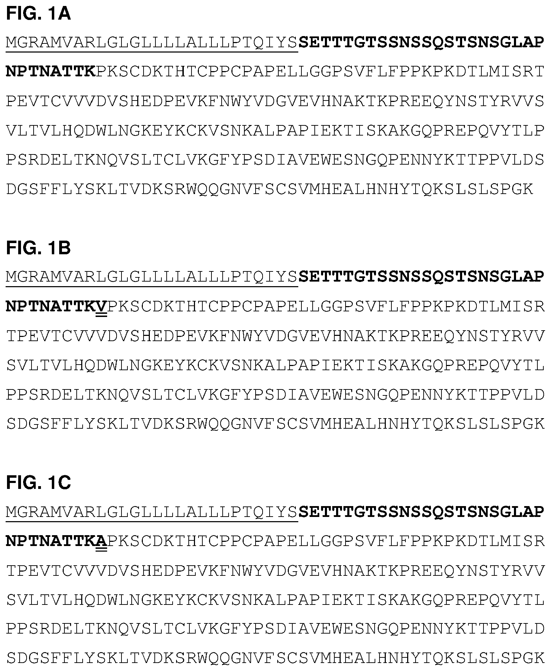

FIG. 1A shows the amino acid composition of the full length CD24 fusion protein, CD24Fc (also referred to herein as CD24Ig) (SEQ ID NO: 5). The underlined 26 amino acids are the signal peptide of CD24 (SEQ ID NO: 4), which are cleaved off during secretion from a cell expressing the protein and thus missing from the processed version of the protein (SEQ ID NO: 6). The bold portion of the sequence is the extracellular domain of the mature CD24 protein used in the fusion protein (SEQ ID NO: 2). The last amino acid (A or V) that is ordinarily present in the mature CD24 protein has been deleted from the construct to avoid immunogenicity. The non-underlined, non-bold letters are the sequence of IgG1 Fc, including the hinge region and CH1 and CH2 domains (SEQ ID NO: 7). FIG. 1B shows the sequence of CD24^{V}Fc (SEQ ID NO: 8), in which the mature human CD24 protein (bold) is the valine polymorphic variant of SEQ ID NO: 1. FIG. 1C shows the sequence of CD24^{A}Fc (SEQ ID NO: 9), in which the mature human CD24 protein (bold) is the alanine polymorphic variant of SEQ ID NO: 1. The various parts of the fusion protein in FIGS. 1B and 1C are marked as in FIG. 1A and the variant valine/alanine amino acid is double underlined.

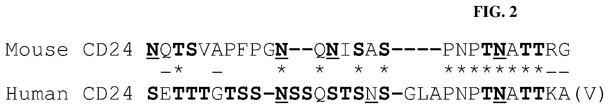

FIG. 2 shows amino acid sequence variations between mature CD24 proteins from mouse (SEQ ID NO: 3) and human (SEQ ID NO: 1). The potential O-glycosylation sites are bolded, and the N-glycosylation sites are underlined.

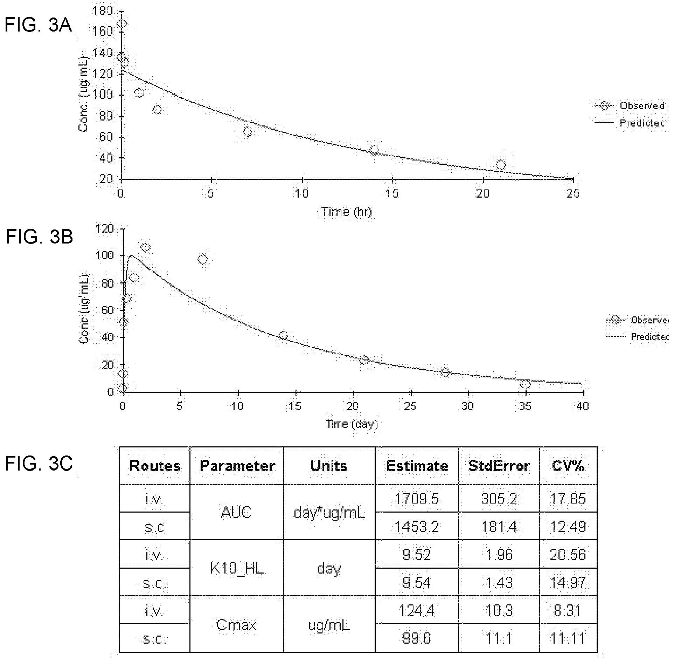

FIG. 3. WinNonlin compartmental modeling analysis of pharmacokenitics of CD24IgG1 (CD24Fc). The opened circles represent the average of 3 mice, and the line is the predicted pharmacokinetic curve. FIG. 3A. i.v. injection of 1 mg CD24IgG1. FIG. 3B. s.c. injection of 1 mg CD24IgG1 (CD24Fc). FIG. 3C. Comparison of the total amounts of antibody in the blood as measured by areas under curve (AUC), half-life and maximal blood concentration. Note that overall, the AUC and Cmax of the s.c. injection is about 80% of i.v. injection, although the difference is not statistically significant.

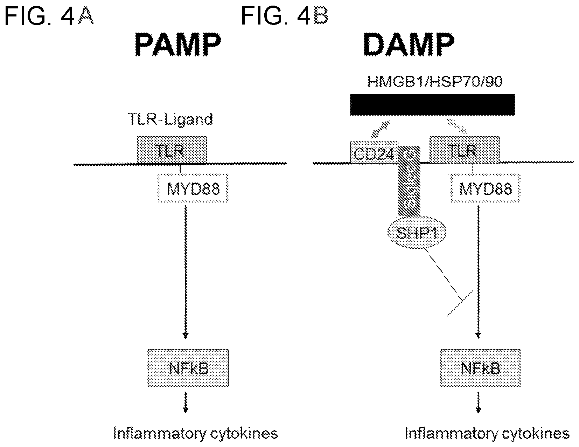

FIG. 4. CD24-Siglec G (10) interaction discriminates between PAMP and DAMP. FIG. 4A. Host response to PAMP was unaffected by CD24-Siglec G(10) interaction. FIG. 4B. CD24-Siglec G (10) interaction represses host response to DAMP, possibly through the Siglec G/10-associated SHP-1.

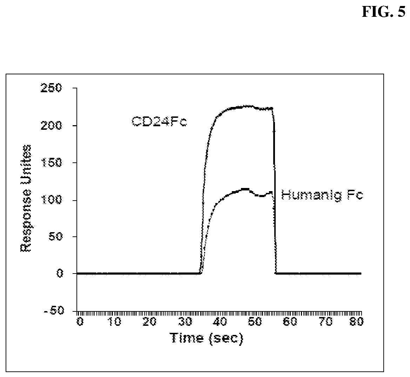

FIG. 5. Binding of CD24Fc with Myelin Associate Glycoprotein (MAG) in vitro

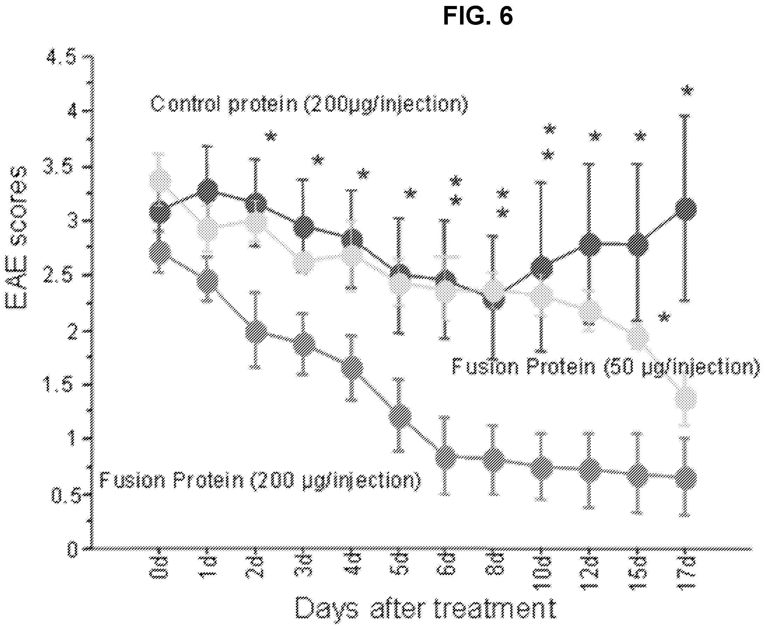

FIG. 6. Therapeutic efficacy of the human CD24 fusion protein in mouse EAE. C57BL/6j mice were immunized with the MOG peptide as described in the legend of FIG. 4. At 14-21 days after immunization, the mice that reached an EAE score between 2.5 and 3.5 were randomly divided into three groups and were treated with 5 injections of either CD24 fusion protein (200 or 50 µg/injection) or control proteins (200 µg/injection) every other day. Data shown are means and S.E. of the clinical score. * P<0.05, ** P<0.01. Overall, a significant therapeutic effect was observed when 200 µg of fusion protein was administered (P=0.0083). Therapeutic effect was observed in two independent experiments with a total of 7-12 mice per group.

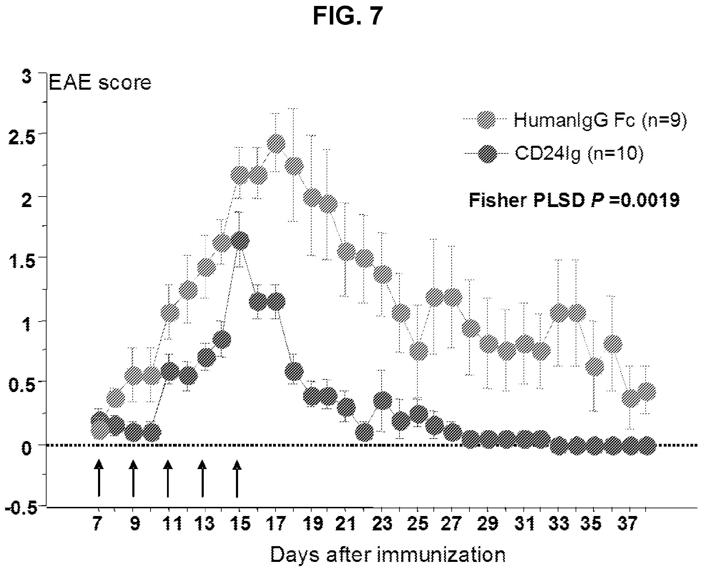

FIG. 7. CD24Fc prevents autoimmune destruction by MOG-primed T cells. Six-week old female C57BL/6 mice were immunized with MOG peptide in CFA on day 0. At day 7 after immunization, when clinical symptoms were absent in the majority of the mice, the mice were treated with either control human IgG Fc or CD24Fc intraperitoneally (200 µg/injection/mouse) every other day for a total of 5 injections, as indicated by the arrows. The mice were inspected daily to score for clinical symptoms. Data shown are mean and SEM with 9 and 10 mice per group. (P=0.0019, Fisher's PLSD test). The therapeutic effect has been reproduced in another experiment.

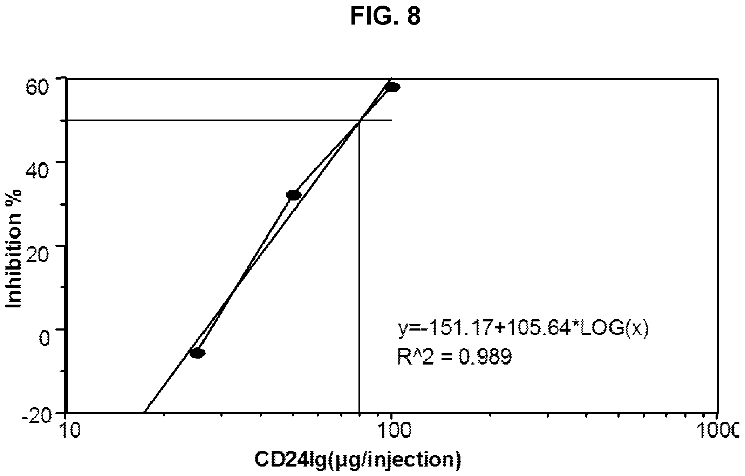

FIG. 8. Identification of the minimal effective dose of CD24Fc in the mouse. C57BL/6 mice were immunized with MOG and treated with CD24Fc as detailed in the legend of FIG. 4. The accumulative disease scores over the first month were used to calculate % of inhibition caused by CD24Fc. The scores of the IgG 1 Fc-treated group were used as a baseline. The logarithmic curve fit was used to calculate the minimal effective dose.

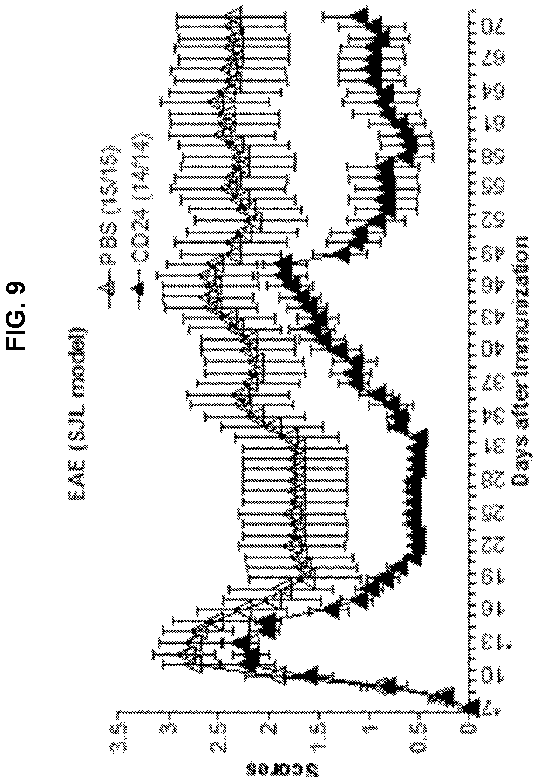

FIG. 9. CD24Fc treatment of relapsing remitting EAE in the SJL model. Six-week old female SJL mice were immunized with PLP peptide in CFA on day 0. At day 7 after immunization, when clinical symptoms were absent in the majority of the mice, the mice were treated with either control human IgG Fc or CD24 Ig intraperitoneally (200 µg/injection/mouse) every other day for a total of 5 injections (days 7, 9, 11, 13 and 15). The mice were inspected daily to score for clinical symptoms. Data shown are mean and SEM with 14 or 15 mice in each group. P=0.0039, Fisher's PLSD test. Therapeutic effect in the SJL model has been repeated 3 times. Since IgGFc exacerbated disease scores in some experiments involving this model (and thus inflated therapeutic effect), GMP-grade vehicle (PBS) was used in this model.

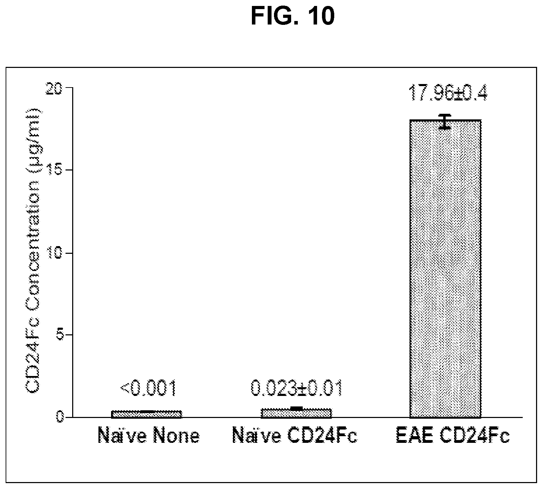

FIG. 10. Concentration of CD24Fc in the CNS of naive and EAE mice. The EAE was induced by the subcutaneous immunization with 200 µg MOG35-55 peptide in Complete Freund's Adjuvant (CFA) containing 400 µg of Mycobacterium tuberculosis into 8-10 weeks female C57BL/6 mice. The immunization was followed by i.v. injection of 200 ng of pertussis toxin immediately and again at 48 hours later. On Day 11 after EAE induction, the mice were administrated i.v. with 1mg CD24Fc. The age-matched naive female C57BL/6 mice also received the same amount of CD24Fc intravenously. 24 hours later, the cerebrospinal fluid (CSF) was harvested from two groups of mice, and the CD24Fc concentrations in the CNS were measured by the sandwich ELISA. In this study, the cerebrospinal fluids from naive mice administrated with PBS were used as negative control.

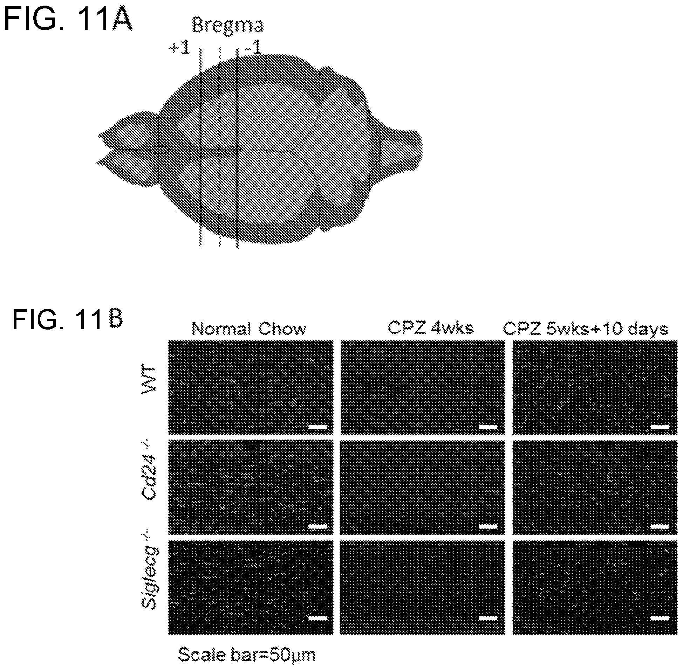

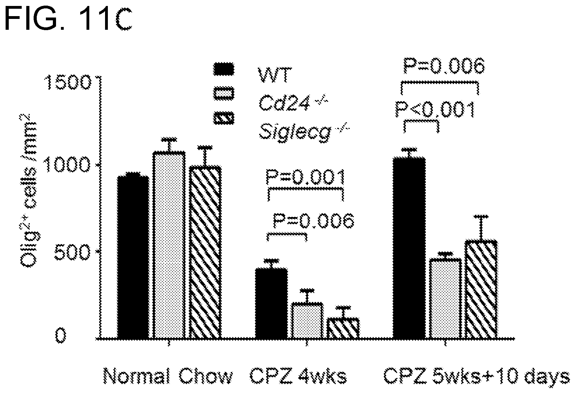

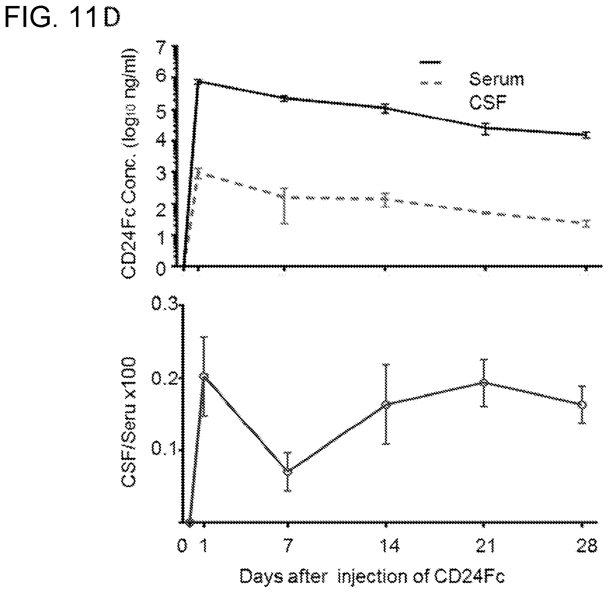

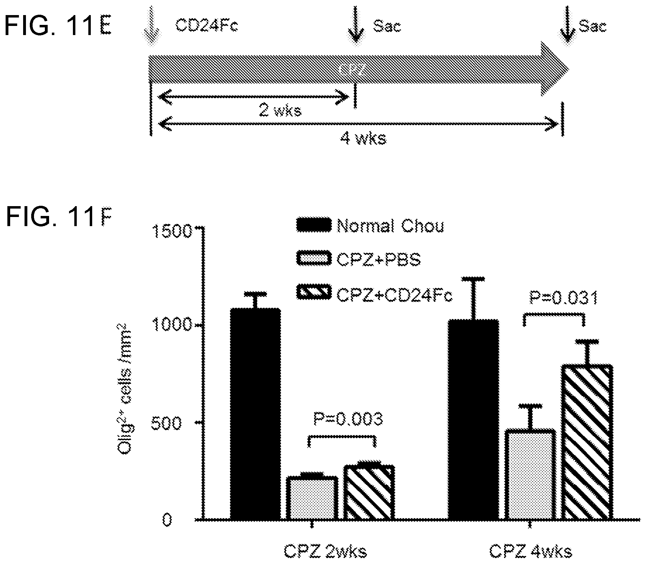

FIG. 11. Targeted mutation of either Cd24 or Siglecg increases susceptibility of oligodendrocytes to Cuprizone (CPZ) treatment. FIG. 11A. Positions of corpus callosum tissue sections analyzed. FIG. 11B. Representative images of oligo-2 staining in Bregma-1 brain regions of WT, Cd24-/- and Siglecg-/- mice. At 8 weeks of age, mice were fed chow containing 0.2% CPZ by weight for 2 to 5 weeks prior to sacrifice. Additional animals were kept on the CPZ-containing diet for 5 weeks and then allowed to recover (removal of CPZ from the diet) for an additional 10 days prior to sacrifice. Controls were maintained in normal chow. Mice were perfused transcardially with phosphate buffered saline (PBS) followed by cold 4% paraformaldehyde in PBS. Brains were removed, fixed overnight in 4% paraformaldehyde, and transferred to cryoprotection solution. Coronal frozen sections (30 µ m, free floating) were cut on a Leica sliding microtome. Free-floating sections were rinsed in PBS and blocked with 5% normal donkey or goat serum for 1 hour at room temperature. Then, sections were incubated 2h at Room temperature with anti-Oligo 2 antibody (EMD Millipore, Cat. No. Ab9610, used at 1:2000 dilution), followed by 1 hour incubation with fluor 555-conjugated donkey anti-rabbit IgG secondary antibodies (Invitrogen, Cat No. A-31572) at room temperature. Subsequently, sections were washed, stained with DAPI, mounted on glass slides, and coverslipped with Fluoromount-G. FIG. 11C. Summary data on the impact on CPZ on the numbers of oligodendrocytes in Bregma -1 region of WT, Cd24-/- and Siglecg-/- brains. Data shown are representative of two independent experiments. P values were determined using student t-test. Data are shown as the mean and standard deviation. FIG. 11D. CD24Fc Concentration detected in CSF and serum after a single systemic administration of CD24Fc (10mg/mouse). These data represent two separated experiments, N=4. Data are shown as the mean and standard deviation. FIG. 11E. Study timelines for therapeutic experiments. FIG. 11F. Numbers of Oligo 2+ cell in Bregma-1 at 2 or 4 wks after a single dose of CD24Fc treatment (10mg/mouse). Data shown are representative of two independent experiments. P value was determined by student t-tests. N=4. Data are shown as the mean and standard deviation. All statistical analyses were performed using GraphPad Prism software. Data are shown as the mean and standard deviation.

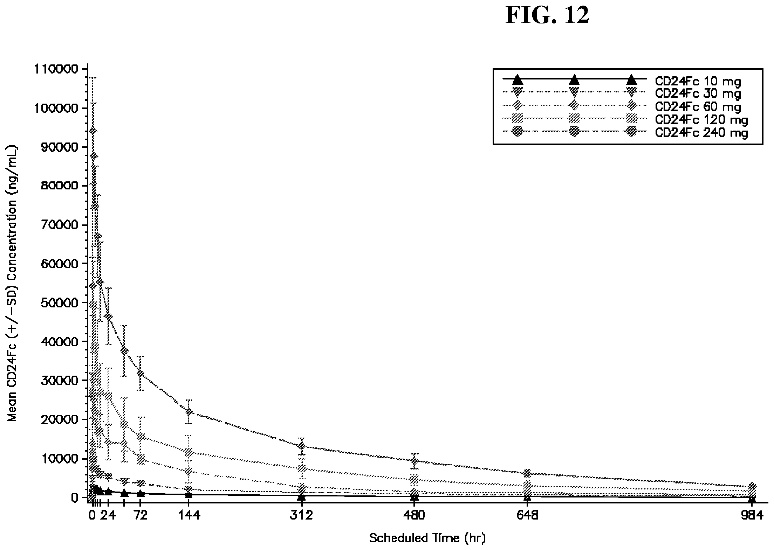

FIG. 12 shows a plot of mean plasma CD24Fc concentration (±SD) by treatment for a PK Evaluable Population in human subjects. PK = pharmacokinetic; SD = standard deviation.

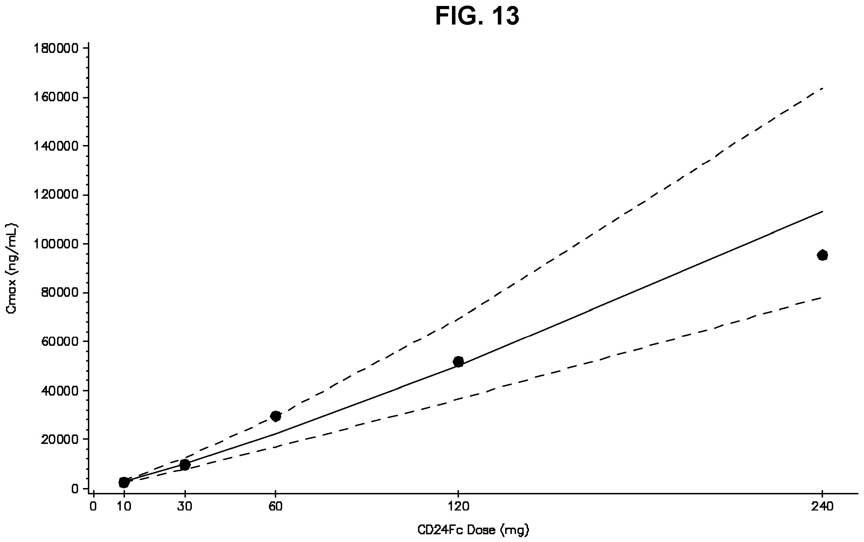

FIG. 13 shows a dose proportionality plot of CD24Fc Cₘₐₓ versus dose for a PK Evaluable Population.

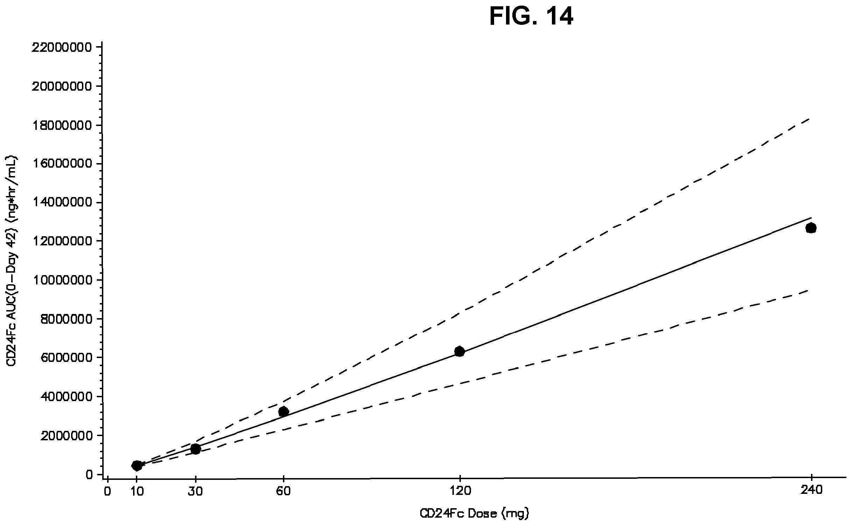

FIG. 14 shows a dose proportionality plot of CD24Fc AUC_{0-42d} versus dose for a PK Evaluable Population.

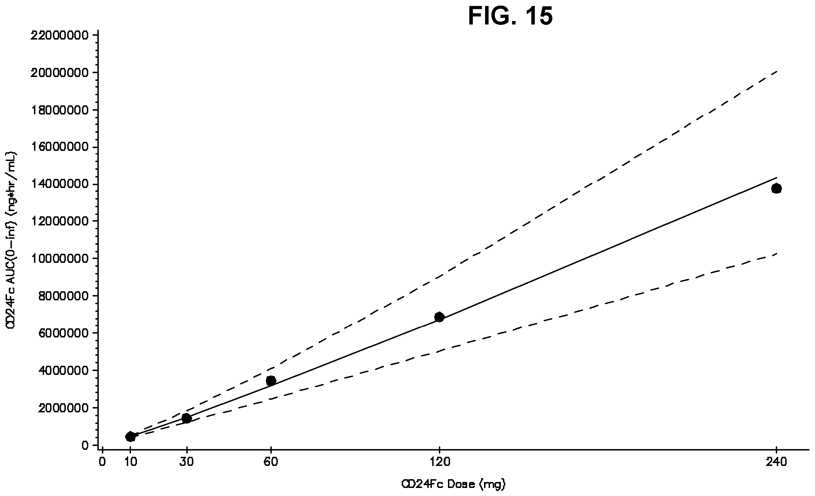

FIG. 15 shows a dose proportionality plot of CD24Fc AUC_{0-inf} versus dose for a PK Evaluable Population.

### DETAILED DESCRIPTION

The inventors have discovered that, surprisingly, a soluble form of CD24 is highly effective for maintaining oligodendrocytes and treating neuro-immune disorders associated with demyelination. The effect may be mediated through damage-associated molecular patterns. Pattern recognition is involved in inflammatory response triggered by both pathogen-associated and tissue damage-associated molecular patterns, respectively called PAMPs and DAMPs. Without being bound by theory, an exacerbated host response to DAMPs may play a part in the pathogenesis of inflammatory and autoimmune disease. DAMPs have been found to promote the production of inflammatory cytokines and autoimmune diseases and in animal models, and inhibitors of DAMPs such as HMGB 1 and HSP90 were consequently found to ameliorate rheumatoid arthritis (RA) (4-6). TLRs, RAGE-R, DNGR (encoded by Clec9A), and Mincle have been shown to be receptors responsible for mediating inflammation initiated by a variety of DAMPs (2, 7-14).

The inventors have demonstrated that CD24-Siglec G interactions discriminate innate immunity to DAMPs from PAMPs (15, 16). Siglec proteins are membrane-associated immunoglobulin (Ig) superfamily members that recognize a variety of sialic acid-containing structures. Most Siglecs have an intra-cellular immune-tyrosine inhibitory motif (ITIM) that associates with SHP-1, -2 and Cbl-b to control key regulators of inflammatory responses. CD24 was identified as the first natural ligand for a Siglec, Siglec G in mouse and Siglec 10 in human (15). Siglec G interacts with sialylated CD24 to suppress the TLR-mediated host response to DAMPs, such as HMGB1, via a SHP-1/2 signaling mechanism (15), which is critical for SLE pathogenesis (2). Siglec G also associates with Cbl to trigger degradation of RIG-I, resulting in the suppression of the type I interferon response (17), a key factor in human lupus pathogenesis.

In addition, the inventors have demonstrated that CD24 negatively regulates host response to cellular DAMPs that are released as a result of tissue or organ damage, and at least two overlapping mechanisms may explain this activity. First, CD24 binds to several DAMPs, including HSP70, HSP90, HMGB 1 and nucleolin and represses host response to these DAMPs. To do this, it is presumed that CD24 may trap the inflammatory stimuli to prevent interaction with their receptors, TLR or RAGE. Second, CD24 through interaction with its receptor Siglec G provides a powerful negative regulation for host response to tissue injuries. To achieve this activity, CD24 may bind and stimulate signaling by Siglec G wherein Siglec G-associated SHP1 triggers the negative regulation. Both mechanisms may act in concert, as mice with targeted mutation of either gene mounted much stronger inflammatory response. In fact, DC cultured from bone marrow from either CD24-/- or Siglec G-/- mice produced higher levels of inflammatory cytokines when stimulated with either HMGB1, HSP70, or HSP90. CD24 is, to the inventors' knowledge, the only inhibitory DAMP receptor capable of shutting down inflammation triggered by DAMPs and no drug is currently available that specifically targets host inflammatory response to tissue injuries. Furthermore, exogenous soluble CD24 protein alleviates DAMP-mediated autoimmune disease in mouse models of rheumatoid arthritis, multiple sclerosis and graft-versus-host diease.

Human CD24 is a small GPI-anchored molecule encoded by an open-reading frame of 240 base pairs in the CD24 gene (28). Of the 80 amino acids, the first 26 constitute the signal peptide, while the last 23 serve as a signal for cleavage to allow for the attachment of the GPI tail. As a result, the mature human CD24 molecule has only 31 amino acids. One of the 31 amino acids is polymorphic among the human population. A C to T transition at nucleotide 170 of the open-reading frame results in the substitution of alanine (A) with valine (V). Since this residue is in the immediate N-terminal to the cleavage site, and since the replacement is non-conservative, these two alleles may be expressed at different efficiencies on the cell surface. Indeed, transfection studies with cDNA demonstrated that the CD24^{v} allele is more efficiently expressed on the cell surface (28). Consistent with this, CD24^{v/v} PBL expressed higher levels of CD24, especially on T cells.

The inventors have discovered that targeted mutation of either Cd24 or Siglecg, which form an axis that selectively regulates innate inflammatory response to DAMPs, protects mice against cuprizone-induced oligodendrocyte loss. The inventors have further discovered that systemic administration of a CD24 protein (CD24Fc), which is known to stimulate Siglec G signaling and suppress inflammatory response in vivo, protects oligodendrocytes against chronic exposure to cuprizone. Therefore, host response to cellular injury actively participates in oligodendrocyte loss, and provides a new approach to maintain oligodendrocytes under pathological conditions.

Accordingly, the methods described herein may be used to provide neuroprotection in order to mitigate, minimize or treat inflammatory and degenerative diseases involving the central nervous system. In particular, the methods described herein may be used treating a neuro-immune disorder including multiple sclerosis (MS), acute disseminated encephalomyelitis (ADEM), neuromyelitis optica spectrum disorder (NMOSD), optic neuritis (ON), and transverse myelitis (TM), which may be acute flaccid myelitis (AFM).

### 1. Definitions.

The terminology used herein is for the purpose of describing particular embodiments only and is not intended to be limiting. As used in the specification and the appended claims, the singular forms "a," "an" and "the" include plural referents unless the context clearly dictates otherwise.

For recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9, and 7.0 are explicitly contemplated.

A "peptide" or "polypeptide" is a linked sequence of amino acids and may be natural, synthetic, or a modification or combination of natural and synthetic.

"Substantially identical" may mean that a first and second amino acid sequence are at least 60%, 65%, 70%, 75%, 80%, 85%, 90%, 95%, 96%, 97%, 98%,or 99% over a region of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, or 300 amino acids.

"Treatment" or "treating," when referring to protection of an animal from a disease, means preventing, suppressing, repressing, or completely eliminating the disease. Preventing the disease involves administering a composition of the present invention to an animal prior to onset of the disease. Suppressing the disease involves administering a composition of the present invention to an animal after induction of the disease but before its clinical appearance. Repressing the disease involves administering a composition of the present invention to an animal after clinical appearance of the disease.

A "variant" may mean a peptide or polypeptide that differs in amino acid sequence by the insertion, deletion, or conservative substitution of amino acids, but retain at least one biological activity. Representative examples of "biological activity" include the ability to bind to a toll-like receptor and to be bound by a specific antibody. Variant may also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art. Kyte et al., J. Mol. Biol. 157:105-132 (1982). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ±2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. U.S. Patent No. 4,554,101, . Substitution of amino acids having similar hydrophilicity values can result in peptides retaining biological activity, for example immunogenicity, as is understood in the art. Substitutions may be performed with amino acids having hydrophilicity values within ±2 of each other. Both the hyrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties.

### 2. CD24

Provided herein is a CD24 protein, which may comprise a mature CD24 or a variant thereof. Mature CD24 corresponds to the extracellular domain (ECD) of CD24. The mature CD24 may be from a human or another mammal. As described above, mature human CD24 protein is 31 amino acids long and ordinarily has a variable alanine (A) or valine (V) residue at its C-terminal end:
SETTTGTSSNSSQSTSNSGLAPNPTNATTK(V/A) (SEQ ID NO: 1)

The C-terminal valine or alanine may be immunogenic and may be omitted from the CD24 protein, which may reduce its immunogenicity. Therefore, the CD24 protein may comprise the amino acid sequence of human CD24 lacking the C-terminal amino acid:
SETTTGTSSNSSQSTSNSGLAPNPTNATTK (SEQ ID NO: 2)

Despite considerable sequence variations in the amino acid sequence of the mature CD24 proteins from mouse and human, they are functionally equivalent, as human CD24Fc has been shown to be active in the mouse. The amino acid sequence of the human CD24 ECD shows some sequence conservation with the mouse protein (39% identity; Genbank accession number NP_033976). However, it is not that surprising that the percent identity is not higher as the CD24 ECD is only 27-31 amino acids in length, depending on the species, and binding to some of its receptor(s), such as Siglec 10/G, is mediated by its sialic acid and/or galactose sugars of the glycoprotein. The amino acid sequence identity between the extracellular domains of the human Siglec-10 (GenBank accession number AF310233) and its murine homolog Siglec-G (GenBank accession number NP_766488) receptor proteins is 63% (FIG. 2). As a result of sequence conservation between mouse and human CD24 primarily in the C-terminus and in the abundance of glycosylation sites, significant variations in the mature CD24 proteins may be tolerated in using the CD24 protein, especially if those variations do not affect the conserved residues in the C-terminus or do not affect the glycosylation sites from either mouse or human CD24. Therefore, the CD24 protein may comprise the amino acid sequence of mature murine CD24:
NQTSVAPFPGNQNISASPNPTNATTRG (SEQ ID NO: 3).

The amino acid sequence of the human CD24 ECD shows more sequence conservation with the cynomolgus monkey protein (52% identity; UniProt accession number UniProtKB - I7GKK1) than with mouse. Again, this is not surprising given that the percent identity is not higher as the ECD is only 29-31 amino acids in length in these species, and the role of sugar residues in binding to its receptor(s). The amino acid sequence of cynomolgous Siglec-10 receptor has not been determined but the amino acid sequence identity between the human and rhesus monkey Siglec-10 (GenBank accession number XP_001116352) proteins is 89%. Therefore, the CD24 protein may also comprise the amino acid sequence of mature cynomolgous (or rhesus) monkey CD24:
TVTTSAPLSSNSPQNTSTTPNPANTTTKA (SEQ ID NO: 10)

The CD24 protein may be soluble. The CD24 protein may further comprise an N-terminal signal peptide, to allow secretion from a cell expressing the protein. The signal peptide sequence may comprise the amino acid sequence MGRAMVARLGLGLLLLALLLPTQIYS (SEQ ID NO: 4). Alternatively, the signal sequence may be any of those that are found on other transmembrane or secreted proteins, or those modified from the existing signal peptides known in the art.

### a. Fusion

The CD24 protein may be fused at its N- or C-terminal end to a protein tag, which may comprise a portion of a mammalian Ig protein, which may be human or mouse or from another species. The portion may comprise an Fc region of the Ig protein. The Fc region may comprise at least one of the hinge region, CH2, CH3, and CH4 domains of the Ig protein. The Ig protein may be human IgG1, IgG2, IgG3, IgG4, or IgA, and the Fc region may comprise the hinge region, and CH2 and CH3 domains of the Ig. The Fc region may comprise the human immunoglobulin G1 (IgG1) isotype SEQ ID NO: 7. The Ig protein may also be IgM, and the Fc region may comprise the hinge region and CH2, CH3, and CH4 domains of IgM. The protein tag may be an affinity tag that aids in the purification of the protein, and/or a solubility-enhancing tag that enhances the solubility and recovery of functional proteins. The protein tag may also increase the valency of the CD24 protein. The protein tag may also comprise GST, His, FLAG, Myc, MBP, NusA, thioredoxin (TRX), small ubiquitin-like modifier (SUMO), ubiquitin (Ub), albumin, or a Camelid Ig. Methods for making fusion proteins and purifying fusion proteins are well known in the art.

Based on preclinical research, for the construction of the fusion protein CD24Fc identified in the examples, the truncated form of native CD24 molecule of 30 amino acids, which lacks the final polymorphic amino acid before the GPI signal cleavage site (that is, a mature CD24 protein having SEQ ID NO: 2), has been used. The mature human CD24 sequence is fused to a human IgG1 Fc domain (SEQ ID NO: 7). The full length CD24Fc fusion protein is provided in SEQ ID NO: 5 (FIG. 1), and the processed version of CD24Fc fusion protein that is secreted from the cell (i.e. lacking the signal sequence which is cleaved off) is provided in SEQ ID NO: 6. Processed polymorphic variants of mature CD24 (that is, mature CD24 protein having SEQ ID NO: 1) fused to IgG1 Fc may comprise SEQ ID NO: 11 or 12.

### b. Production

The CD24 protein may be heavily glycosylated, and may be involved in functions of CD24 such as costimulation of immune cells and interaction with a damage-associated molecular pattern molecule (DAMP). The CD24 protein may be prepared using a eukaryotic expression system. The expression system may entail expression from a vector in mammalian cells, such as Chinese Hamster Ovary (CHO) cells. The system may also be a viral vector, such as a replication-defective retroviral vector that may be used to infect eukaryotic cells. The CD24 protein may also be produced from a stable cell line that expresses the CD24 protein from a vector or a portion of a vector that has been integrated into the cellular genome. The stable cell line may express the CD24 protein from an integrated replication-defective retroviral vector. The expression system may be GPEx^{™}.

### c. Pharmaceutical composition

The CD24 protein may be contained in a pharmaceutical composition, which may comprise a pharmaceutically acceptable amount of the CD24 protein. The pharmaceutical composition may comprise a pharmaceutically acceptable carrier. The pharmaceutical composition may comprise a solvent, which may keep the CD24 protein stable over an extended period. The solvent may be PBS, which may keep the CD24 protein stable for at least 66 months at -20°C (-15~-25°C). The solvent may be capable of accommodating the CD24 protein in combination with another drug.

The pharmaceutical composition may be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

The pharmaceutical composition may also be formulated as a depot preparation, which may be administered by implantation or by intramuscular injection. The composition may be formulated with suitable polymeric or hydrophobic materials (as an emulsion in an acceptable oil, for example), ion exchange resins, or as sparingly soluble derivatives (as a sparingly soluble salt, for example). A formulation for subcutaneous injection may be particularly relevant for an indication like lupus and its associated manifestations and complications.

### d. Dosage

The dose of the CD24 protein may ultimately be determined through a clinical trial to determine a dose with acceptable toxicity and clinical efficacy. The initial clinical dose may be estimated through pharmacokinetics and toxicity studies in rodents and non-human primates. The dose of the CD24 protein may be 0.01 mg/kg to 1000mg/kg, and may be 1 to 500 mg/kg, depending on the desired degree of oligodendrocyte maintenance or on the neuro-immune disorder being treated, and the route of administration. The CD24 protein may be administered by intravenous infusion or subcutaneous, intramural (that is, within the wall of a cavity or organ), or intraperitoneal injection, and the dose may be 10-1000 mg, 10-500 mg, 10-240 mg, 10-120 mg, or 10, 30, 60, 120, or 240 mg, where the subject is a human.

### 3. Methods of treatment

Provided herein is a method of maintaining oligodendrocytes by administering a CD24 protein described herein to a subject in need thereof. Further provided herein is a method of treating a demyelinating disorder, by administering the CD24 protein to a subject in need thereof. The CD24 protein may be administered to a subject with or at risk of developing the demyelinating disorder. Oligodendrocytes may be maintained in the central nervous system (CNS) of the subject by enhancing or improving oligodendrocyte myelinating activity.

The demyelinating disorder is neuromyelitis optica spectrum disorder (NMOSD), optic neuritis (ON), or transverse myelitis (TM), which may be acute flaccid myelitis (AFM). The CD24 protein may also be used to mitigate, reduce or treat the neuro-immune disorder.

### a. Administration

The route of administration of the pharmaceutical composition may be parenteral. Parenteral administration includes, but is not limited to, intravenous, intraarterial, intraperitoneal, subcutaneous, intramuscular, intrathecal, intraarticular, and direct injection. The pharmaceutical composition may be administered to a human patient, cat, dog, large animal, or an avian. The composition may be administered 1, 2, 3, 4, 5, 6, 7, 8, 9, 10, 11, or 12 times per day.

### b. Combination treatment

The CD24 proteins described herein may be combined with another treatment used to promote remyelination or which promotes the conversion of oligodendrocyte precursor cells in oligodendrocytes. Examples of such treatments include anti-Lingo-1, NDC-1308, anti-EphrinB3, clemastine fumarate, fluorosamine, and fingolimod. LINGO-1 is a protein produced by the CNS that prevents immature cells from becoming oligodendrocytes and anti-Lingo (opicinumab) can block the action of LINGO-1 allowing these cells to mature into oligodendrocytes. NDC-1308 is designed to repair the myelin sheath of demyelinated nerve fibers and is specifically targeted to heal motor neuron damage. EphrinB3 is a membrane-bound signaling protein that blocks the remyelination of damaged neurons in MS and so anti-Ephrin is designed to reverse this blockage. Fluorosamine was seen to boost remyelination in mice by preventing the synthesis of chondroitin sulfate proteoglycans and by promoting oligodendrocyte function. Similarly, fingolimod (GILENYA), a drug approved for patients with relapsing multiple sclerosis to prevent neuro inflammation, may also help these patients by directly enhancing nerve regeneration and increasing myelination in a way that is partly independent of its anti-inflammatory properties.

The CD24 protein may be administered simultaneously or metronomically with other treatments. The term "simultaneous" or "simultaneously" as used herein, means that the CD24 protein and other treatment be administered within 48 hours, preferably 24 hours, more preferably 12 hours, yet more preferably 6 hours, and most preferably 3 hours or less, of each other. The term "metronomically" as used herein means the administration of the agent at times different from the other treatment and at a certain frequency relative to repeat administration.

The CD24 protein may be administered at any point prior to another treatment including about 120 hr, 118 hr, 116 hr, 114 hr, 112 hr, 110 hr, 108 hr, 106 hr, 104 hr, 102 hr, 100 hr, 98 hr, 96 hr, 94 hr, 92 hr, 90 hr, 88 hr, 86 hr, 84 hr, 82 hr, 80 hr, 78 hr, 76 hr, 74 hr, 72 hr, 70 hr, 68 hr, 66 hr, 64 hr, 62 hr, 60 hr, 58 hr, 56 hr, 54 hr, 52 hr, 50hr, 48 hr, 46 hr, 44 hr, 42 hr, 40 hr, 38 hr, 36 hr, 34 hr, 32 hr, 30 hr, 28 hr, 26 hr, 24 hr, 22 hr, 20 hr, 18 hr, 16 hr, 14 hr, 12 hr, 10 hr, 8 hr, 6 hr, 4 hr, 3 hr, 2 hr, 1 hr, 55 mins., 50 mins., 45 mins., 40 mins., 35 mins., 30 mins., 25 mins., 20 mins., 15 mins, 10 mins, 9 mins, 8 mins, 7 mins., 6 mins., 5 mins., 4 mins., 3 mins, 2 mins, and 1 mins. The CD24 protein may be administered at any point prior to a second treatment of the CD24 protein including about 120 hr, 118 hr, 116 hr, 114 hr, 112 hr, 110 hr, 108 hr, 106 hr, 104 hr, 102 hr, 100 hr, 98 hr, 96 hr, 94 hr, 92 hr, 90 hr, 88 hr, 86 hr, 84 hr, 82 hr, 80 hr, 78 hr, 76 hr, 74 hr, 72 hr, 70 hr, 68 hr, 66 hr, 64 hr, 62 hr, 60 hr, 58 hr, 56 hr, 54 hr, 52 hr, 50hr, 48 hr, 46 hr, 44 hr, 42 hr, 40 hr, 38 hr, 36 hr, 34 hr, 32 hr, 30 hr, 28 hr, 26 hr, 24 hr, 22 hr, 20 hr, 18 hr, 16 hr, 14 hr, 12 hr, 10 hr, 8 hr, 6 hr, 4 hr, 3 hr, 2 hr, 1 hr, 55 mins., 50 mins., 45 mins., 40 mins., 35 mins., 30 mins., 25 mins., 20 mins., 15 mins., 10 mins., 9 mins., 8 mins., 7 mins., 6 mins., 5 mins., 4 mins., 3 mins, 2 mins, and 1 mins.

The CD24 protein may be administered at any point after another treatment including about 1min, 2 mins., 3 mins., 4 mins., 5 mins., 6 mins., 7 mins., 8 mins., 9 mins., 10 mins., 15 mins., 20 mins., 25 mins., 30 mins., 35 mins., 40 mins., 45 mins., 50 mins., 55 mins., 1 hr, 2 hr, 3 hr, 4 hr, 6 hr, 8 hr, 10 hr, 12 hr, 14 hr, 16 hr, 18 hr, 20 hr, 22 hr, 24 hr, 26 hr, 28 hr, 30 hr, 32 hr, 34 hr, 36 hr, 38 hr, 40 hr, 42 hr, 44 hr, 46 hr, 48 hr, 50 hr, 52 hr, 54 hr, 56 hr, 58 hr, 60 hr, 62 hr, 64 hr, 66 hr, 68 hr, 70 hr, 72 hr, 74 hr, 76 hr, 78 hr, 80 hr, 82 hr, 84 hr, 86 hr, 88 hr, 90 hr, 92 hr, 94 hr, 96 hr, 98 hr, 100 hr, 102 hr, 104 hr, 106 hr, 108 hr, 110 hr, 112 hr, 114 hr, 116 hr, 118 hr, and 120 hr. The CD24 protein may be administered at any point prior after a previous CD24 treatment including about 120 hr, 118 hr, 116 hr, 114 hr, 112 hr, 110 hr, 108 hr, 106 hr, 104 hr, 102 hr, 100 hr, 98 hr, 96 hr, 94 hr, 92 hr, 90 hr, 88 hr, 86 hr, 84 hr, 82 hr, 80 hr, 78 hr, 76 hr, 74 hr, 72 hr, 70 hr, 68 hr, 66 hr, 64 hr, 62 hr, 60 hr, 58 hr, 56 hr, 54 hr, 52 hr, 50hr, 48 hr, 46 hr, 44 hr, 42 hr, 40 hr, 38 hr, 36 hr, 34 hr, 32 hr, 30 hr, 28 hr, 26 hr, 24 hr, 22 hr, 20 hr, 18 hr, 16 hr, 14 hr, 12 hr, 10 hr, 8 hr, 6 hr, 4 hr, 3 hr, 2 hr, 1 hr, 55 mins., 50 mins., 45 mins., 40 mins., 35 mins., 30 mins., 25 mins., 20 mins., 15 mins., 10 mins., 9 mins., 8 mins., 7 mins., 6 mins., 5 mins., 4 mins., 3 mins, 2 mins, and 1 mins.

### Example 1

### CD24 pharmacokinetics in mice

1 mg of CD24Fc (CD24Fc) was injected into naive C57BL/6 mice and collected blood samples at different timepoints (5 min, 1 hr, 4 hrs, 24 hrs, 48 hrs, 7 days, 14 days and 21 days) with 3 mice in each timepoint. The sera were diluted 1:100 and the levels of CD24Fc was detected using a sandwich ELISA using purified anti-human CD24 (3.3 µg/ml) as the capturing antibody and peroxidase conjugated goat anti-human IgG Fc (5 µg/ml) as the detecting antibodies. As shown in FIG. 3A. The decay curve of CD24Fc revealed a typical biphase decay of the protein. The first biodistribution phase had a half-life of 12.4 hours. The second phase follows a model of first-order elimination from the central compartment. The half-life for the second phase was 9.54 days, which is similar to that of antibodies in vivo. These data suggest that the fusion protein is very stable in the blood stream. In another study in which the fusion protein was injected subcutaneously, an almost identical half-life of 9.52 days was observed (FIG. 3B). More importantly, while it took approximately 48 hours for the CD24Fc to reach peak levels in the blood, the total amount of the fusion protein in the blood, as measured by AUC, was substantially the same by either route of injection. Thus, from a therapeutic point of view, different route of injection should not affect the therapeutic effect of the drug. This observation greatly simplified the experimental design for primate toxicity and clinical trials.

### Example 2

### CD24-Siglec 10 interaction in host response to tissue injuries

Nearly two decades ago, Matzinger proposed what was popularly called danger theory. In essence, she argued that the immune system is turned on when it senses the dangers in the host. Although the nature of danger was not well defined at the time, it has been determined that necrosis is associated with the release of intracellular components such as HMGB1 and Heat-shock proteins, which were called DAMP, for danger-associated molecular patterns. DAMP were found to promote production of inflammatory cytokines and autoimmune diseases. In animal models, inhibitors of HMGB 1 and HSP90 were found to ameliorate RA. The involvement of DAMP raised the prospect that negative regulation for host response to DAMP can be explored for RA therapy.

Using acetaminophen-induced liver necrosis and ensuring inflammation, it was observed that through interaction Siglec G, CD24 provides a powerful negative regulation for host response to tissue injuries. CD24 is a GPI anchored molecule that is broadly expressed in hematopoietic cells and other tissue stem cells. Genetic analysis of a variety of autoimmune disease in human, including multiple sclerosis, systemic lupus erythromatosus, RA, and giant cell arthritis, showed significant association between CD24 polymorphism and risk of autoimmune diseases. Siglec G is a member of I-lectin family, defined by their ability to recognize sialic acid containing structure. Siglec G recognized sialic acid containing structure on CD24 and negatively regulates production of inflammatory cytokines by dendritic cells. In terms of its ability to interact with CD24, human Siglec 10 and mouse Siglec G are functionally equivalent. However, it is unclear if there is a one-to-one correlation between mouse and human homologues. Although the mechanism remains to be full elucidated, it is plausible that SiglecG-associated SHP1 may be involved in the negative regulation. These data, led to a new model in which CD24-Siglec G/10 interaction may play a critical in discrimination pathogen-associated molecular pattern (PAMP) from DAMP (FIG. 4).

At least two overlapping mechanisms may explain the function of CD24. First, by binding to a variety of DAMP, CD24 may trap the inflammatory stimuli to prevent their interaction with TLR or RAGE. This notion is supported by observations that CD24 is associated with several DAMP molecules, including HSP70, 90, HMGB 1 and nucleolin. Second, perhaps after associated with DAMP, CD24 may stimulate signaling by Siglec G. Both mechanisms may act in concert as mice with targeted mutation of either gene mounted much stronger inflammatory response. In fact, DC cultured from bone marrow from either CD24-/- or Siglec G/- mice produced much higher inflammatory cytokines when stimulated with either HMGB 1, HSP70, or HSP90. In contrast, no effect were found in their response to PAMP, such as LPS and PolyLC. These data not only provided a mechanism for the innate immune system to distinguish pathogen from tissue injury, but also suggest that CD24 and Siglec G as potential therapeutic targets for diseases associated with tissue injuries.

### Example 3

### Binding of CD24Fc with Myelin Associate Glycoprotein (MAG) In Vitro

Myelin Associate Glycoprotein (MAG), also known as Siglec-4, is a member of the Siglec family that recognizes sialic acid-containing structures and is expressed on the axons of neurons and functions as a negative regulator for axon outgrowth and neuron-regeneration. MAG protein is conserved between mice and humans allowing the testing of CD24Fc in mouse models. In order to confirm binding of CD24Fc to MAG, recombinant MAG-Fc fusion protein comprising the extracellular domain of rat MAG fused by means of a polypeptide linker to the carboxyl-terminal Fc region of human IgG1 was used (Sigma product number M 5063). The extracellular domain of human MAG (Genbank accession number NP_002352) and rat MAG (Genbank accession number NP_058886) are 96% identical.

The interaction of CD24Fc with MAG was detected by Biacore surface plasmon resonance (SPR) assay. Briefly, both CD24Fc and human IgG Fc control were biotinylated and immobilized on sensor chip that was pre-immobilized with streptavidin. After the preparation of sensor chip surface, 10µl MAG-Fc at different concentrations (1, 6 and 24 µM) was injected sequentially into the flow channels. Each injection was followed by 10 min dissociation by washing with HBS as eluent. The binding of MAG-Fc to the CD24Fc or human IgG Fc immobilized on the sensor surface generated a response which is proportional to the bound mass. The response is measured as resonance units (RU), which reflected the signal changes recorded after the injection of different concentrations of MAG-Fc.'

The real-time graph of response units against time demonstrated that, compared with human IgG Fc, the CD24Fc has a higher binding for MAG-Fc. The specific binding is presented in dose-dependent manner (FIG. 5). Therefore, CD24Fc may potentially block the function of MAG during axon growth and neuron-regeneration.

### Example 4

### Therapeutic Efficacy of CD24Fc in Preclinical Models

Treatment of animals with ongoing clinical signs of EAE.

C57BL/6 mice 8-12 weeks of age were immunized subcutaneously with 200 µg MOG (peptide 35-55 of rat origin (MEVGWYRSPFSRVVHLYRNGK) in CFA (400 µg of Mycobacterium tuberculosis per milliliter) in a total volume of 100 µL. Each mouse received 200 ng of pertussis toxin (List Biological, Campbell, California, USA) in 200 µL PBS in the tail vein immediately after the immunization and again 48 hours later. The mice were observed every other day and were scored on a scale of 0-5 with gradations of 0.5 for intermediate scores: 0, no clinical signs; 1, loss of tail tone; 2, wobbly gait; 3, hind limb paralysis; 4, hind and fore limb paralysis; and 5, death. On days 14-21 after immunization, the mice with clinical scores of 2.5-3.5 were randomly divided into two groups and treated with either control IgG1 Fc or CD24Fc. Five injections of 200 □g/mice every other day were given intraperitoneally (i.p.). As shown in FIG. 6, CD24Fc had a significant and dose-dependent therapeutic effect.

Treatment of animals with primed autoreactive T cells.

To test whether CD24Fc can prevent the destruction of autoreactive T cells, we treated immunized mice on day 7 after immunization. At this point, MOG-reactive T cells could be found in the lymph nodes, but no clinical symptoms could be observed. At day 7 after immunization, the immunized mice were divided into 2 groups of 9 mice per group and treated with either control immunoglobulin (Ig) or the CD24Fc fusion protein intraperitoneally (200 µg/injection/mouse) every other day for a total of 5 injections (days 7, 9, 11, 13 and 15). The mice were inspected daily to score for clinical symptoms as described above.

As shown in FIG. 7, at 2 days after administration of either control IgG or the CD24Fc, the group treated with the CD24Fc showed a delay in the development of clinical symptoms as compared to the control group. When all data from the two groups were considered, two statistical analyses revealed highly significant differences (P=0.0019, Fisher's PLSD test). These results demonstrate that the clinical symptoms of EAE can be delayed and lessened with treatment with CD24Fc fusion protein.

To determine the minimal effective dose, we injected 100, 50 and 25 µg /injection of CD24Fc into the MOG immunized mice. We used a reduction of 50% in the accumulative disease score over a 1 month period as the endpoint. As shown in FIG. 8, a dose-dependent inhibition was observed. The dose response curve fit well with the logarithmic curve, which indicated that 80 µg /injection yielded a 50% reduction in the accumulative disease score.

Treatment of relapsing remitting EAE in the SJL model.

The EAE in C57BL/6 mice has an acute clinical course which differs from the chronic course that presents in the overwhelming majority of MS patients. A large proportion of MS patients have a relapsing remitting clinical course, which is best mimicked in the SJL model of EAE. We therefore tested the therapeutic potential in the SJL model. The treatment regimen was the same as the above experiment. Briefly, at day 7 after immunization, the immunized mice were divided into 2 groups of 14 or 15 mice per group and treated with either vehicle control or the CD24Fc fusion protein of the present invention intraperitoneally (200 µg/injection/mouse) every other day for a total of 5 injections (days 7, 9, 11, 13 and 15). As shown in FIG. 9, treatment of SJL mice with CD24Fc resulted in a substantial reduction in clinical scores (P=0.0039, Fisher's PLSD test).

### Example 5

### The Biodistribution of CD24Fc to the Central Nerve System in Mice with Ongoing Experimental Autoimmune Encephalomyelitis.

The Central Nerve System (CNS) is the target organ of the immunotherapy for multiple sclerosis. The objective of this study is to investigate the biodistribution of CD24Fc in the CNS of naive mice and mice with on-going experimental autoimmune encephalomyelitis (EAE).

The results in FIG. 10 show that 24 hours after CD24Fc administration, naive mice had very low but detectable levels of CD24Fc (23 ng/ml). However, in the mice with on-going EAE, the CD24Fc level was about 18µg/ml, which is more than 780-fold higher than in the CNS of the naive mice. The increase is extremely significant (P<0.001, the non-parametric Mann-Whitney T test). The level of CD24Fc in the CNS is well within the effective range based on *in vitro* inhibition assays. Therefore, CD24Fc may gain access to the CNS and provide therapeutic effect locally in the CNS of patients with multiple sclerosis (MS).

### Example 6

### Use of CD24 for treating demyelinating diseases and neuroprotection

This Example demonstrates that a CD24 protein can be used to treat demyelinating diseases and neuro-immune diseases, provide neuroprotection, and maintain oligodendrocytes. Chronic exposure of cuprizone is a classical model to induce oligodendrocyte loss in rodents (5). Several groups have reported on a critical role for inflammation in loss of oligodendrocytes. However, the nature of initiating signal for oligodendrocyte loss has not been clearly delineated. Janeway has proposed that innate immunity is initiated primarily through pattern recognition of microbial products called pathogen-associated molecular patterns or PAMPs (6, 7). Subsequently, Matzinger proposed danger signal, which is later termed DAMPs by others, as the key to initiate innate immune response (8). Studies by the inventors have shown that inflammation induced by PAMPs and DAMPs can be discriminated by CD24-Siglec G interaction as targeted mutation of either gene selectively enhanced host response to DAMPs without affecting that to PAMPs (9, 10). Here, following the inventors' insight into potential new uses for CD24, the potential contribution of innate immune response to DAMPs was explored by evaluating the impact of targeted mutations of the Cd24 or Siglecg genes in oligodendrocyte survival after chronic cuprizone treatment.

Age- and gender matched WT, Cd24^{-/-} and Siglecg^{-/-} mice (9, 10) were treated with CPZ for 4 weeks, and euthanized mice at 4 weeks after treatment. To monitor recovery of oligodendrocytes, the mice were first treated for 5 weeks and allowed to recover for 10 days with normal chow for 10 days and then euthanized for analysis. The Bregma-1 region (FIG. 11A) were sectioned and analyzed for the number of oligodendrocytes by immunofluorescence staining using anti-Oligo-2 antibody that specifically binds to all cells in the oligodendrocyte lineage. Representative images are shown in FIG. 11B, while summary data are shown in FIG. 11C. As shown in FIG. 11B, all three strains of mice had comparable number of oligodendrocytes prior to CPZ treatment. These data suggest that Cd24 and Siglec G are not involvement in the development of oligodendrocytes in this region. However, targeted mutations of either Cd24 or Siglecg gene resulted in a more severe depletion of oligodendrocytes at 4 weeks after CPZ treatment. Moreover, while the oligodendrocyte numbers were fully restored after 10 days of recovery period in WT mice, only partial recoveries were observed in Cd24^{-/-} and Siglecg^{-/-} brains (FIG. 11C). These data demonstrate that the CD24-Siglec G axis protects oligodendrocytes against CPZ toxicities.

To determine if Siglec G agonist CD24Fc (9, 10) (described in U.S. Patent Publication No. 20130231464,) can be administrated systemically to protect oligodendrocytes, it was first determined whether CD24Fc can be delivered into the CNS through systemic administration. 10 mg of CD24Fc were injected into 8 week old mice intraperitoneally. Serum and CSF were collected at day 1, 7, 14, 21 and 28 days, and the amounts of CD24Fc were measured by Sandwich ELISA. As shown in FIG. 11D, CD24Fc can be found in the CSF throughout the observation period of 4 weeks, with levels that largely parallel to those observed in the plasma. The CSF CD24Fc levels are approximately 0.2-0.3% of the plasma levels. The levels found at 4 weeks were higher than IC50 of CD24Fc in assays for inhibiting production of inflammatory cytokines by macrophages.

The protective effect of CD24Fc was analyzed at 2 and 4 weeks after CPZ treatment, based on the in situ analysis of the Oligo2+ oligodendrocytes. The oligodendrocyte numbers Bregma-1 positions of corpus callosum (FIG. 11E) were analyzed because this region suffered more pathological damage during CPZ treatment. It was observed that CD24Fc treatment significantly increased the number of oligodendrocytes at both 2 and 4 weeks after CPZ-treatment (FIG. 11F).

Since loss of oligodendrocytes is usually associated with reduced remyelination, it was further investigated whether targeted mutation of either CD24 of Siglecg genes caused accelerated demyelination. Surprisingly, more severe demyelination was not observed in either strain of mice. These data suggested that improved oligodendrocyte survival may not be sufficient to induce remyelination.

Taken together, the data described herein demonstrate that the CD24-Siglec G axis protects oligodendrocyte against CPZ killing, and thus implicate host response to DAMPs as an unrecognized culprit for oligodendrocyte loss in the CPZ model. The new insight into pathogenesis of oligodendrocyte loss suggests a new approach to promote oligodendrocyte survival *in vivo.* Consistent with this notion, it has been shown that systemic administration of CD24Fc, which binds to DAMPs (9, 10) and works as an agonist for Siglec G (9, 10) is sufficient to protect oligodendrocytes against killing by CPZ. Since loss of oligodendrocytes has been implicated in multiple neurodegenerative diseases (1-4), the data suggest that host response in cellular injuries may be explored for therapeutic development of these intractable neurological diseases. Finally, it should be noted that CD24 is also known as a costimulatory molecule for activation of T cells (11), including autoreactive T cells (12), and intrahepatic CD4 T cells (13). Correspondingly, CD24Fc was also shown to inhibit autoimmune diseases (14). Since CD24Fc phenocopies the genetic defect of the Cd24 gene in the autoimmune diseases (12, 14), it likely works through a different mechanism as an antagonist.

### Example 7

### CD24 pharmacokinetics in humans

This example shows an analysis of the pharmacokinetics of a CD24 protein in humans. This was derived from a Phase I, randomized, double-blind, placebo-controlled, single ascending dose study to assess the safety, tolerability, and PK of CD24Fc in healthy male and female adult subjects. A total of 40 subjects in 5 cohorts of 8 subjects each were enrolled in this study. Six of the 8 subjects in each cohort received study drug and 2 subjects received placebo (0.9% sodium chloride, saline). The first cohort was dosed with 10 mg. Succeeding cohorts received 30 mg, 60 mg, 120 mg, and 240 mg of CD24Fc or matching placebo and were dosed at least 3 weeks apart to allow for review of safety and tolerability data for each prior cohort. Administration of the next higher dose to a new cohort of subjects was permitted only if adequate safety and tolerability had been demonstrated.

In each cohort, the initial 2 subjects were 1 study drug recipient and 1 placebo recipient on Day 1. The 3rd to 5th and 6th to 8th subjects were dosed after Day 7 (a minimum of 24 hours apart between the subgroups). Each subject was dosed at least 1 hour apart in the same subgroup. If necessary, dosing of the rest of subjects was delayed pending review of any significant safety issues that may have arisen during the post-dose period involving the first or second subgroups in that cohort. The subsequent cohort was dosed at least 3 weeks after the prior cohort.

### Screening Period:

The Screening Visit (Visit 1) occured up to 21 days prior to the beginning of the active treatment period. After providing informed consent, subjects underwent screening procedures for eligibility.

### Treatment Period:

Subjects were admitted to the Clinical Pharmacology Unit (CPU) on Day -1 (Visit 2), and the randomized treatment period began on Day 1 following a 10-hour minimum overnight fast. Subjects were randomly assigned to treatment with CD24Fc or placebo as a single dose. Subjects remained confined until the morning of Day 4.

### Follow-up:

All subjects returned to the CPU on Day 7, Day 14, Day 21, Day 28, and Day 42 (±1 day) for follow-up visits (Visit 3, Visit 4, Visit 5, Visit 6, and Visit 7). Visit 7 was the final visit for all subjects.

Duration of Treatment: The total study duration for each subject was up to 63 days. Single-dose administration occurred on Day 1.

### Number of Subjects:

Planned: 40 subjects
Screened: 224 subjects
Randomized: 40 subjects
Completed: 39 subjects
Discontinued: 1 subject

Diagnosis and Main Criteria for Inclusion: The population for this study was healthy males and females between the ages of 18 and 55 years, inclusive, with a body mass index between 18 kg/m² and 30 kg/m², inclusive.

### Investigational Product and Comparator Information:

CD24Fc: single dose of 10 mg, 30 mg, 60 mg, 120 mg, or 240 mg administered via IV infusion; lot number: 09MM-036. CD24Fc was a fully humanized fusion protein consisting of the mature sequence of human CD24 and the fragment crystallizable region of human immunoglobulin G1 (IgG1Fc). CD24Fc was supplied as a sterile, clear, colorless, preservative-free, aqueous solution for IV administration. CD24Fc was formulated as single dose injection solution, at a concentration of 10 mg/mL and a pH of 7.2. Each CD24Fc vial contained 160 mg of CD24Fc, 5.3 mg of sodium chloride, 32.6 mg of sodium phosphate dibasic heptahydrate, and 140 mg of sodium phosphate monobasic monohydrate in 16 mL ± 0.2 mL of CD24Fc. CD24Fc was supplied in clear borosilicate glass vials with chlorobutyl rubber stoppers and aluminum flip-off seals.

Matching placebo (0.9% sodium chloride, saline) administered via IV infusion; lot numbers: P296855, P311852, P300715, P315952.

The intent-to-treat (ITT) Population consisted of all subjects who received at least 1 dose of the study drug. The ITT Population was the primary analysis population for subject information and safety evaluation.

Clinical laboratory evaluations (chemistry, hematology, and urinalysis) were summarized by treatment and visit. Change from baseline was also summarized. Vital signs (blood pressure, heart rate, respiratory rate, and temperature) were summarized by treatment and time point. Change from baseline was also summarized. All physical examination data were listed. Electrocardiogram parameters and the change from baseline were summarized. Overall interpretations were listed.

### Plasma CD24Fc Concentration

As shown in FIG. 12, the mean plasma concentration of CD24Fc increased proportionally to the dose of CD24Fc administered. For all dose groups except 120 mg, the maximum mean plasma concentration of CD24Fc was reached at 1 hour post-dose. The maximum mean plasma concentration of CD24Fc for the 120 mg group was reached at 2 hours post-dose. By Day 42 (984 hours), the mean plasma concentration of CD24Fc for all groups had decreased to between 2% and 4% of the maximum mean plasma concentration.

Table 1 summarizes the plasma CD24Fc PK parameters by treatment for the PK Evaluable Population.

**Table 1 Summary of Plasma CD24Fc Pharmacokinetic Parameters by Treatment - PK Evaluable Population**

| **Parameter Statistic** | **CD24Fc 10 mg (N=6)** | **CD24Fc 30 mg (N=6)** | **CD24Fc 60 mg (N=6)** | **CD24Fc 120 mg (N=6)** | **CD24Fc 240 mg (N=6)** |
|---|---|---|---|---|---|
| Cₘₐₓ (ng/mL) | | | | | |
| n | 6 | 6 | 6 | 6 | 6 |
| Mean (SD) | 2495 (576) | 9735 (1715) | 30 083 (7179) | 52 435 (9910) | 95 865 (10 734) |
| CV% | 23.1 | 17.6 | 23.9 | 18.9 | 11.2 |
| Median | 2371 | 9218 | 29 026 | 50 401 | 93 206 |
| Min, Max | 1,967, 3,390 | 8,583, 13,086 | 22,557, 42,628 | 40,434, 65,704 | 81,296, 110,110 |
| Geometric mean | 2,442 | 9,625 | 29,424 | 51,666 | 95,365 |
| Geometric CV% | 22.8 | 16.1 | 23.0 | 19.0 | 11.2 |
| AUC_{0-42d} (ng*hr/mL) | | | | | |
| n | 6 | 6 | 6 | 6 | 6 |
| Mean (SD) | 423,061 (99,615) | 1,282,430 (88,798) | 3,226,255 (702,862) | 6,541,501 (2,190,944) | 12,704,705 (1,918,596) |
| CV% | 23.5 | 6.9 | 21.8 | 33.5 | 15.1 |
| Median | 434,043 | 1,302,719 | 3,124,933 | 5,785,142 | 12,563,426 |
| Min, Max | 291,020, 528,079 | 1,175,733, 1,403,024 | 2,487,550, 4,139,748 | 4,485,193, 9,415,266 | 10,466,635, 15,693,606 |
| Geometric mean | 412,795 | 1,279,851 | 3,163,252 | 6,249,552 | 12,586,731 |
| Geometric CV% | 25.0 | 7.0 | 22.0 | 33.8 | 15.0 |
| AUC_{0-inf} (ng*hr/mL) | | | | | |
| n | 6 | 6 | 6 | 6 | 6 |
| Mean (SD) | 462,260 (116,040) | 1,434,464 (131,316) | 3,497,196 (705,653) | 7,198,196 (2,458,320) | 13,861,796 (1,962,780) |
| CV% | 25.1 | 9.2 | 20.2 | 34.2 | 14.2 |
| Median | 470,426 | 1,422,205 | 3,519,732 | 6,463,665 | 13,713,034 |
| Min, Max | 310,956, 596,599 | 1,281,715, 1,650,503 | 2,703,655, 4,309,023 | 4,910,640, 10,479,940 | 11,822,988, 17,175,236 |
| Geometric mean | 449,583 | 1,429,578 | 3,437,036 | 6,862,129 | 13,750,972 |
| Geometric CV% | 26.7 | 9.0 | 20.7 | 34.6 | 13.8 |
| Tₘₐₓ (hr) | | | | | |
| n | 6 | 6 | 6 | 6 | 6 |
| Mean (SD) | 1.15 (0.42) | 1.17 (0.41) | 1.01 (0.01) | 1.34 (0.51) | 1.33 (0.52) |
| CV% | 36.1 | 35.0 | 1.2 | 38.0 | 38.7 |
| Median | 1.00 | 1.00 | 1.00 | 1.03 | 1.00 |
| Min, Max | 0.92, 2.00 | 1.00, 2.00 | 1.00, 1.03 | 1.00, 2.00 | 1.00, 2.00 |
| t½ (hr) | | | | | |
| n | 6 | 6 | 6 | 6 | 6 |
| Mean (SD) | 280.83 (22.37) | 327.10 (41.32) | 279.82 (65.59) | 286.45 (23.38) | 285.33 (24.33) |
| CV% | 8.0 | 12.6 | 23.4 | 8.2 | 8.5 |
| Median | 279.61 | 317.23 | 264.69 | 290.76 | 287.74 |
| Min, Max | 258.87, 321.26 | 289.82, 394.24 | 210.18, 362.46 | 243.89, 309.26 | 249.24, 322.26 |
| AUCextr (%) | | | | | |
| n | 6 | 6 | 6 | 6 | 6 |
| Mean (SD) | 7.61 (2.14) | 10.44 (2.94) | 7.88 (4.26) | 8.92 (1.94) | 8.46 (1.99) |
| CV% | 28.1 | 28.2 | 54.0 | 21.8 | 23.5 |
| Median | 7.16 | 10.01 | 6.35 | 9.27 | 8.45 |
| Min, Max | 5.46, 11.47 | 7.10, 15.05 | 3.92, 14.48 | 5.49, 10.99 | 5.56, 11.50 |
| CL (L/hr) | | | | | |
| n | 6 | 6 | 6 | 6 | 6 |
| Mean (SD) | 0.0229 (0.0061) | 0.0211 (0.0019) | 0.0178 (0.0036) | 0.0183 (0.0058) | 0.0176 (0.0023) |
| CV% | 26.7 | 8.8 | 20.5 | 31.7 | 13.3 |
| Median | 0.0216 | 0.0211 | 0.0173 | 0.0191 | 0.0175 |
| Min, Max | 0.0168, 0.0322 | 0.0182, 0.0234 | 0.0139, 0.0222 | 0.0115, 0.0244 | 0.0140, 0.0203 |
| Vd (L) | | | | | |
| n | 6 | 6 | 6 | 6 | 6 |
| Mean (SD) | 9.153 (1.943) | 9.867 (0.804) | 7.289 (2.592) | 7.491 (2.202) | 7.276 (1.426) |
| CV% | 21.2 | 8.1 | 35.6 | 29.4 | 19.6 |
| Median | 8.507 | 10.007 | 7.486 | 7.691 | 7.151 |
| Min, Max | 7.326, 12.010 | 8.771, 10.958 | 4.222, 11.139 | 4.933, 9.974 | 5.814, 9.438 |
| AUC_{0-42d} = area under the concentration-time curve from time 0 to 42 days; AUC_{0-inf} = area under the concentration-time curve extrapolated from time 0 to infinity; AUCₑₓₜᵣ = percentage of AUC_{0-inf} that was due to extrapolation from the time of | | | | | |
| the last measurable concentration, per subject, to infinity; CL = total body clearance; Cₘₐₓ = maximum observed plasma drug concentration; CV% = coefficient of variation; Min = minimum; Max = maximum; SD = standard deviation; t_{½} = terminal elimination half-life; Tₘₐₓ = time of maximum observed plasma drug concentration; V_{d} = volume of distribution. | | | | | |

### Plasma CD24Fc Dose Proportionality Analysis

FIG. 13 shows a dose proportionality plot of CD24Fc Cₘₐₓ versus dose for the PK Evaluable Population. FIG. 14 shows a dose proportionality plot of CD24Fc AUC_{0-42d} versus dose for the PK Evaluable Population. FIG. 15 shows a dose proportionality plot of CD24Fc AUC_{0-inf} versus dose for the PK Evaluable Population. Table 2 shows a power analysis of dose proportionality.

**Table 2 Power Analysis of Dose Proportionality: Plasma CD24Fc Pharmacokinetic Parameters - PK Evaluable Population**

| **Parameter Statistic** | **CD24Fc 10 mg (N=6)** | **CD24Fc 30 mg (N=6)** | **CD24Fc 60 mg (N=6)** | **CD24Fc 120 mg (N=6)** | **CD24Fc 240 mg (N=6)** | **Dose Proportionality** | | |
|---|---|---|---|---|---|---|---|---|
| | | | | | | **Slope Estimate** | **Standard Error** | **90% CI** |
| Cₘₐₓ (ng/mL) | | | | | | 1.172 | 0.040 | (1.105, 1.240) |
| Geometric mean | 2,441.8 | 9,624.9 | 29,424.4 | 51,666.4 | 95,364.9 | | | |
| Geometric CV% | 22.8 | 16.1 | 23.0 | 19.0 | 11.2 | | | |
| AUC_{0-42d} (ng*hr/mL) | | | | | | 1.088 | 0.036 | (1.027, 1.148) |
| Geometric mean | 412,794.8 | 1,279,850.8 | 3,163,251.7 | 6,249,551.9 | 12,586,731.3 | | | |
| Geometric CV% | 25.0 | 7.0 | 22.0 | 33.8 | 15.0 | | | |
| AUC_{0-inf} (ng*hr/mL) | | | | | | 1.087 | 0.036 | (1.026, 1.148) |
| Geometric mean | 449,583.5 | 1,429,577.5 | 3,437,035.6 | 6,862,128.7 | 13,750,972.4 | | | |
| Geometric CV% | 26.7 | 9.0 | 20.7 | 34.6 | 13.8 | | | |
| Geometric CV% = 100^{∗}sqrt(exp(SD²)-1), where SD was the standard deviation of the log-transformed data. The power model was fitted by restricted maximum likelihood, regressing the log-transformed PK parameter on log transformed dose. Both the intercept and slope were fitted as fixed effects. Dose proportionality was not rejected if the 90% CI lies within (0.8, 1.25). | | | | | | | | |
| AUC_{0-42d} = area under the concentration-time curve from time 0 to 42 days; AUC_{0-inf} = area under the concentration-time curve extrapolated from time 0 to infinity; C1 = confidence interval; Cₘₐₓ - maximum observed plasma drug concentration; CV% = coefficient of variation; PK = pharmacokinetic; SD = standard deviation. | | | | | | | | |

The Cₘₐₓ slope estimate was 1.172 with a 90% CI of 1.105 to 1.240. The AUC_{0-42d} slope estimate was 1.088 with a 90% CI of 1.027 to 1.148. The AUC_{0-inf} slope estimate was 1.087 with a 90% CI of 1.026 to 1.1.

### Pharmacokinetic Conclusions

The Cₘₐₓ and AUCs of plasma CD24Fc increased proportionally to the doses administered in mouse, monkey and human. The plasma CD24Fc reached Tₘₐₓ between 1.01 and 1.34 hours. The t_{½} of plasma CD24Fc ranged between 280.83 and 327.10 hours.

### References

1 Nave, K. A. & Trapp, B. D. Axon-glial signaling and the glial support of axon function. Annu. Rev. Neurosci. 31, 535-561, doi:10.1146/annurev.neuro.30.051606.094309 (2008).
2 Rivera, A., Vanzuli, I., Arellano, J. J. & Butt, A. Decreased Regenerative Capacity of Oligodendrocyte Progenitor Cells (NG2-Glia) in the Ageing Brain: A Vicious Cycle of Synaptic Dysfunction, Myelin Loss and Neuronal Disruption? Current Alzheimer research 13, 413-418 (2016).
3 Cai, Z. & Xiao, M. Oligodendrocytes and Alzheimer's disease. Int. J. Neurosci. 126, 97-104, doi:10.3109/00207454.2015.1025778 (2016).
4 McTigue, D. M. & Tripathi, R. B. The life, death, and replacement of oligodendrocytes in the adult CNS. J. Neurochem. 107, 1-19, doi:10.1111/j.1471-4159.2008.05570.x (2008).
5 Blakemore, W. F. Observations on oligodendrocyte degeneration, the resolution of status spongiosus and remyelination in cuprizone intoxication in mice. J. Neurocytol. 1, 413-426 (1972).
6 Janeway, C. A. Approaching the asymptote? Evolution and revolution in immunology. Cold Spring Harb. Symp. Quant. Biol. 54, 1-13 (1989).
7 Liu, Y. & Janeway, C. A., Jr. Microbial induction of co-stimulatory activity for CD4 T-cell growth. Int. Immunol. 3, 323-332 (1991).
8 Matzinger, P. Tolerance, danger, and the extended family. Annu. Rev. Immunol. 12, 991-1045 (1994).
9 Chen, G. Y. et al. Amelioration of sepsis by inhibiting sialidase-mediated disruption of the CD24-SiglecG interaction. Not. Biotechnol. 29, 428-435, doi:nbt.1846 [pii] 10.1038/nbt.1846 (2011).
10 Chen, G. Y., Tang, J., Zheng, P. & Liu, Y. CD24 and Siglec-10 Selectively Repress Tissue Damage-Induced Immune Responses. Science 323, 1722-1725 (2009).
11 Fang, X., Zheng, P., Tang, J. & Liu, Y. CD24: from A to Z. Cellular & molecular immunology 7, 100-103 (2010).
12 Bai, X. F. et al. CD24 Controls Expansion and Persistence of Autoreactive T Cells in the Central Nervous System during Experimental Autoimmune Encephalomyelitis. J. Exp. Med. 200, 447-458 (2004).
13 Zheng, C., Yin, S., Yang, Y., Yu, Y. & Xie, X. CD24 aggravates acute liver injury in autoimmune hepatitis by promoting IFN-gamma production by CD4+ T cells. Cellular & molecular immunology, doi:10.1038/cmi.2016.57 (2017).
14 Bai, X. F. et al. The heat-stable antigen determines pathogenicity of self-reactive T cells in experimental autoimmune encephalomyelitis. J. Clin. Invest. 105, 1227-1232 (2000).

## Claims

1. A CD24 protein for use in treating a demyelinating disorder in a subject, wherein the demyelinating disorder is selected from the group consisting of an Alzheimer's disease, a Parkinson's disease, neuromyelitis optica spectrum disorder, optic neuritis, transverse myelitis, and acute flaccid myelitis.

2. The CD24 protein for use of claim 1, wherein the demyelinating disorder is selected from the group consisting of an Alzheimer's disease and a Parkinson's disease.

3. The CD24 protein for use of claim 1 or 2, wherein the CD24 protein maintains oligodendrocytes.

4. The CD24 protein for use of any of the preceding claims, wherein another agent that promotes the conversion of oligodendrocytes or myelin production by oligodendrocytes is used for treating the subject.

5. The CD24 protein for use of any of the preceding claims, wherein the CD24 protein comprises a mature human CD24 polypeptide comprising the sequence set forth in SEQ ID NO: 1 or 2.

6. The CD24 protein for use of any of the preceding claims, wherein the CD24 protein comprises a Fc region of a mammalian Ig protein, wherein the Fc region is fused at the N-terminus or C-terminus of the CD24 protein.

7. The CD24 protein for use of claim 6, wherein the Fc region comprises a hinge region and CH2 and CH3 domains of a human Ig protein selected from the group consisting of IgG1, IgG2, IgG3, IgG4, and IgA.

8. The CD24 protein for use of claim 6, wherein the Fc region comprises a hinge region and CH2, CH3 and CH4 domains of human IgM.

9. The CD24 protein for use of any one of claims 1-7, wherein the CD24 protein comprises the amino acid sequence set forth in SEQ ID NO: 6, 11, or 12.

10. The CD24 protein for use of any one of the preceding claims, wherein the CD24 protein is soluble.

11. The CD24 protein for use of any of the preceding claims, wherein the CD24 protein is glycosylated

12. The CD24 protein for use of any one of the preceding claims, wherein the CD24 protein is produced using a eukaryotic protein expression system.

13. The CD24 protein for use of claim 12, wherein the expression system comprises a vector contained in a Chinese Hamster Ovary cell line or a replication-defective retroviral vector.

14. The CD24 protein for use of claim 13, wherein the replication-defective retroviral vector is stably integrated into the genome of a eukaryotic cell.

15. The CD24 protein for use of any one of claims 1-7 and 9-14, wherein the amino acid sequence of the CD24 protein consists of the sequence set forth in SEQ ID NO: 6, 11, or 12.

## Patentansprüche

1. CD24-Protein zur Verwendung bei der Behandlung einer demyelinisierenden Störung bei einer Person, wobei die demyelinisierende Störung aus der Gruppe ausgewählt ist, die aus einer Alzheimer-Krankheit, einer Parkinson-Krankheit, einer Neuromyelitis-optica-Spektrum-Störung, einer Optikusneuritis, einer transventrikulären Myelitis und einer akuten schlaffen Myelitis besteht.

2. CD24-Protein zur Verwendung nach Anspruch 1, wobei die demyelinisierende Störung aus der Gruppe ausgewählt ist, die aus einer Alzheimer-Krankheit und einer Parkinson-Krankheit besteht.

3. CD24-Protein zur Verwendung nach Anspruch 1 oder 2, wobei das CD24-Protein Oligodendrozyten erhält.

4. CD24-Protein zur Verwendung nach einem der vorhergehenden Ansprüche, wobei ein anderes, die Umwandlung von Oligodendrozyten oder die Myelinherstellung durch Oligodendrozyten förderndes Mittel zur Behandlung der Person verwendet wird.

5. CD24-Protein zur Verwendung nach einem der vorhergehenden Ansprüche, wobei das CD24-Protein ein reifes menschliches CD24-Polypeptid umfasst, das die in SEQ ID NO: 1 oder 2 dargelegte Sequenz umfasst.

6. CD24-Protein zur Verwendung nach einem der vorhergehenden Ansprüche, wobei das CD24-Protein eine Fc-Region eines Säugetier-Ig-Proteins umfasst, wobei die Fc-Region an den N-Terminus oder C-Terminus des CD24-Proteins fusioniert ist.

7. CD24-Protein zur Verwendung nach Anspruch 6, wobei die Fc-Region eine Hinge-Region und CH2- und CH3-Domänen eines menschlichen Ig-Proteins, ausgewählt aus der Gruppe bestehend aus IgG1, IgG2, IgG3, IgG4 und IgA, umfasst.

8. CD24-Protein zur Verwendung nach Anspruch 6, wobei die Fc-Region eine Hinge-Region und CH2-, CH3- und CH4-Domänen des menschlichen IgM umfasst.

9. CD24-Protein zur Verwendung nach einem der Ansprüche 1-7, wobei das CD24-Protein die in SEQ ID NO: 6, 11 oder 12 dargestellte Aminosäuresequenz umfasst.

10. CD24-Protein zur Verwendung nach einem der vorhergehenden Ansprüche, wobei das CD24-Protein löslich ist.

11. CD24-Protein zur Verwendung nach einem der vorhergehenden Ansprüche, wobei das CD24-Protein glykosyliert ist

12. CD24-Protein zur Verwendung nach einem der vorhergehenden Ansprüche, wobei das CD24-Protein unter Verwendung eines eukaryotischen Proteinexpressionssystems hergestellt wird.

13. CD24-Protein zur Verwendung nach Anspruch 12, wobei das Expressionssystem einen Vektor umfasst, der in einer Ovarialzellinie des chinesischen Hamsters oder einem replikationsdefekten retroviralen Vektor enthalten ist.

14. CD24-Protein zur Verwendung nach Anspruch 13, wobei der replikationsdefekte retrovirale Vektor stabil in das Genom einer eukaryotischen Zelle integriert ist.

15. CD24-Protein zur Verwendung nach einem der Ansprüche 1 bis 7 und 9 bis 14, wobei die Aminosäuresequenz des CD24-Proteins aus der in SEQ ID NO: 6, 11 oder 12 dargelegten Sequenz besteht.

## Revendications

1. Protéine CD24 destinée à être utilisée dans le traitement d'un trouble démyélinisant chez un sujet, le trouble démyélinisant étant choisi dans le groupe consistant en une maladie d'Alzheimer, une maladie de Parkinson, un trouble du spectre de la neuromyélite optique, une névrite optique, une myélite transverse et une myélite flasque aiguë.

2. Protéine CD24 destinée à être utilisée selon la revendication 1, dans laquelle le trouble démyélinisant est choisi dans le groupe consistant en une maladie d'Alzheimer et une maladie de Parkinson

3. Protéine CD24 destinée à être utilisée selon la revendication 1 ou 2, dans laquelle la protéine CD24 maintient les oligodendrocytes.

4. Protéine CD24 destinée à être utilisée selon l'une quelconque des revendications précédentes, dans laquelle un autre agent qui favorise la conversion des oligodendrocytes ou la production de myéline par les oligodendrocytes est utilisé pour traiter le sujet.

5. Protéine CD24 destinée à être utilisée selon l'une quelconque des revendications précédentes, dans laquelle la protéine CD24 comprend un polypeptide CD24 humain mature comprenant la séquence indiquée dans SEQ ID NO: 1 ou 2.

6. Protéine CD24 destinée à être utilisée selon l'une quelconque des revendications précédentes, dans laquelle la protéine CD24 comprend une région Fc d'une protéine IG mammifère, la région Fc étant fusionnée à l'extrémité N-terminale ou C-terminale de la protéine CD24.

7. Protéine CD24 destinée à être utilisée selon la revendication 6, dans laquelle la région Fc comprend une région charnière et des domaines CH2 et CH3 d'une protéine Ig humaine choisie dans le groupe constitué de IgG1, IgG2, IgG3, IgG4 et IgA.

8. Protéine CD24 destinée à être utilisée selon la revendication 6, dans laquelle la région Fc comprend une région charnière et les domaines CH2, CH3 et CH4 de l'IgM humaine.

9. Protéine CD24 destinée à être utilisée selon l'une quelconque des revendications 1 à 7, dans laquelle la protéine CD24 comprend la séquence d'acide aminé indiquée en SEQ ID NO: 6, 11 ou 12.

10. Protéine CD24 destinée à être utilisée selon l'une quelconque des revendications précédentes, dans laquelle la protéine CD24 est soluble.

11. Protéine CD24 destinée à être utilisée selon l'une quelconque des revendications précédentes, dans laquelle la protéine CD24 est glycosylée.

12. Protéine CD24 destinée à être utilisée selon l'une quelconque des revendications précédentes, dans laquelle la protéine CD24 est produite en utilisant un système d'expression de protéines eucaryotes.

13. Protéine CD24 destinée à être utilisée selon la revendication 12, dans laquelle le système d'expression comprend un vecteur contenu dans une lignée cellulaire d'ovaires de hamster chinois ou un vecteur rétroviral défectueux de réplication.

14. Protéine CD24 destinée à être utilisée selon la revendication 13, où le vecteur rétroviral défectueux de replication est intégré de manière stable dans le génome d'une cellule eucaryote.

15. Protéine CD24 destinée à être utilisée selon l'une quelconque des revendications 1 à 7 et 9 à 14, dans laquelle la séquence d'acide aminé de la protéine CD24 est constituée de la séquence indiquée en SEQ ID NO: 6, 11 ou 12.
